(19) Europäisches Patentamt
European Patent Office
Office européen des brevets

(11) Veröffentlichungsnummer: **0 047 496**
**A1**

(12) # EUROPÄISCHE PATENTANMELDUNG

(21) Anmeldenummer: **81106883.2**

(22) Anmeldetag: **03.09.81**

(51) Int. Cl.³: **C 07 C 143/833**, C 07 C 139/00, A 61 K 31/64

(30) Priorität: **10.09.80 DE 3034004**

(43) Veröffentlichungstag der Anmeldung: **17.03.82 Patentblatt 82/11**

(84) Benannte Vertragsstaaten: **DE FR GB IT**

(71) Anmelder: **HOECHST AKTIENGESELLSCHAFT, Postfach 80 03 20, D-6230 Frankfurt/Main 80 (DE)**

(72) Erfinder: **Hitzel, Volker, Dr., Kantstrasse 1b, D-6238 Hofheim am Taunus (DE)**
Erfinder: **Weyer, Rudi, Dr., Johann-Strauss-Strasse 43, D-6233 Kelkheim (Taunus) (DE)**
Erfinder: **Geisen, Karl, Dr., Jahnstrasse 43, D-6000 Frankfurt am Main (DE)**

(54) **Sulfonylharnstoffe, Verfahren zu ihrer Herstellung, pharmazeutische Präparate auf Basis dieser Verbindungen und ihre Verwendung.**

(57) Sulfonylharnstoffe der Formel

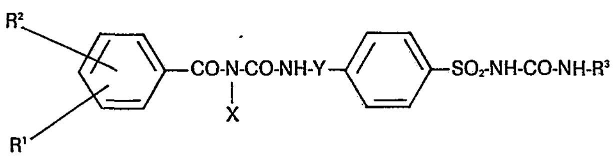

worin $R^1$, $R^2$, $R^3$, X und Y die angegebenen Bedeutungen haben, sowie deren physiologisch verträglichen Salze, ihre Verwendung bei der Behandlung des Diabetes und pharmazeutische Präparate auf Basis dieser Verbindungen.

EP 0 047 496 A1

ACTORUM AG

0047496

# Sulfonylharnstoffe, Verfahren zu ihrer Herstellung, pharmazeutische Präparate auf Basis dieser Verbindungen und ihre Verwendung

Die Erfindung betrifft Sulfonylharnstoffe der Formel

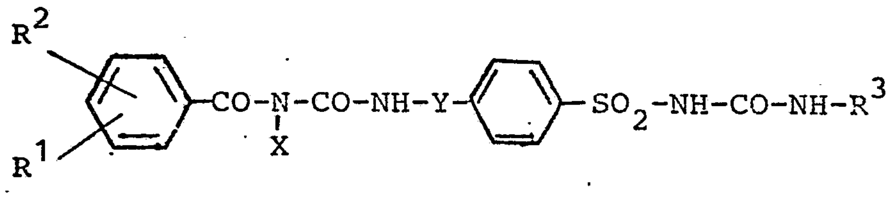

die als Substanz oder in Form ihrer physiologisch verträglichen Salze blutzuckersenkende Eigenschaften besitzen und sich durch eine starke Senkung des Blutzuckerspiegels auszeichnen und daher als Arzneimittel verwendet werden können.

In der Formel bedeuten:

$R^1$, $R^2$ Wasserstoff, Alkyl, Alkoxy mit jeweils 1 bis 3 C-Atomen im Alkylteil, oder Halogen, wobei $R^1$ und $R^2$ gleich oder verschieden sein können,

X Wasserstoff, Alkyl mit 1 - 3 C-Atomen,
Y Alkylen mit 2 - 3 C-Atomen,
$R^3$ Alkyl von 3 bis 8 C-Atomen, Cycloalkyl, Alkylcycloalkyl, Dialkylcycloalkyl, Cycloalkylalkyl, Cycloalkenyl, Alkylcycloalkenyl mit jeweils 4 - 9 C-Atomen, Methylcyclopentylmethyl Cyclohexenylmethyl, Chlorcyclohexyl, Methoxycyclohexyl, Bicycloheptyl, Bicycloheptenyl, Bicycloheptylmethyl, Bicycloheptenylmethyl, Bicyclooctyl, Nortricyclyl, Adamantyl oder Benzyl.

In der allgemeinen Formel bedeutet Y vorzugsweise $-CH_2-CH_2-$, $-\underset{CH_3}{\underset{|}{CH}}-CH_2-$, wobei die $-CH_2-CH_2-$Gruppe besonders bevorzugt ist. $R^3$ ist vorzugsweise Butyl, Cyclopentyl, Cyclohexyl, 4-Methyl-cyclohexyl. Als bicyclische Reste kommen beispielsweise in Frage: 2,5-Endomethylencyclohexyl, 2,5-Endomethylencyclohexylmethyl sowie die entsprechenden ungesättigten Reste und der 2,5-Endoäthylencyclohexylrest. Halogen bedeutet vorzugsweise Chlor. Für den Substituenten X kommt insbesondere die Bedeutung Wasserstoff in Betracht. Der Substituent $R^1$ befindet sich vorzugsweise in 2-Stellung und der Substituent $R^2$ in 5-Stellung zur CO-Gruppierung, wobei für den Substituenten $R^1$ Methoxy und für den Substituenten $R^2$ Methyl und Chlor besonders bevorzugt sind.

Die Erfindung betrifft ferner Verfahren zur Herstellung dieser Sulfonylharnstoffe, pharmazeutische Präparate, die diese enthalten oder aus ihnen bestehen sowie die Verwendung zur Behandlung des Diabetes.

Die Verfahren zur Herstellung sind dadurch gekennzeichnet, daß man

a) mit der Gruppe

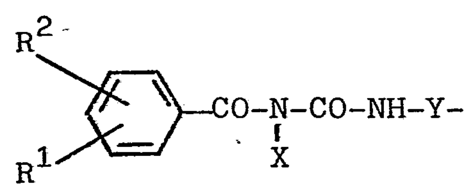

in 4-Stellung substituierte Benzolsulfonylcarbaminsäurederivate mit einem Amin $R^3-NH_2$ oder dessen Salzen umsetzt oder Sulfonamide der Formel

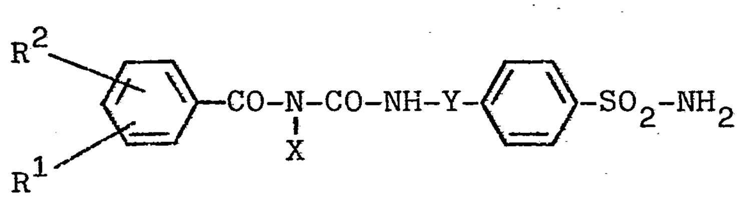

oder deren Salze mit einem $R^3$-substituierten Carbaminsäurederivat umsetzt,

b) mit der Gruppe

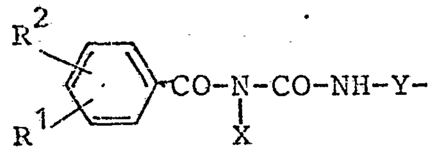

substituierte Benzolsulfonyl-isoharnstoffäther, -isothioharnstoffäther, -parabansäuren oder-halogenameisensäureamidine spaltet,

c) in

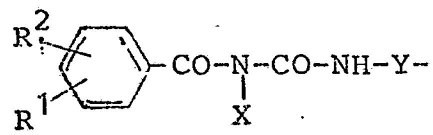

substituierten Benzolsulfonylthioharnstoffen das Schwefelatom durch Sauerstoff ersetzt,

d) entsprechende Benzolsulfinyl- oder -sulfenyl-harnstoffe oxydiert,

e) in Benzolsulfonylharnstoffe der Formel

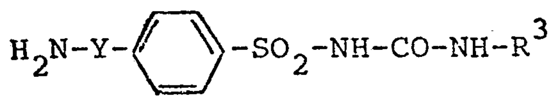

gegebenenfalls stufenweise den Rest

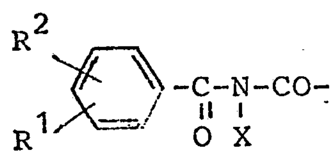

einführt,

f) entsprechend substituierte Benzolsulfonylhalogenide mit $R^3$-substituierten Harnstoffen oder deren Alkalisalzen umsetzt oder entsprechend substituierte Benzolsulfinsäure-halogenide oder, in Gegenwart von sauren Kondensationsmitteln, auch entsprechend substituierte Sulfinsäuren oder deren Alkalisalze, mit N-$R^3$-N'-hydroxy-harnstoff umsetzt

und die Reaktionsprodukte gegebenenfalls zur Salzbildung mit alkalischen Mitteln behandelt.

Die in Verfahren a) erwähnten Benzolsulfonylcarbaminsäurederivate können Benzolsulfonylcarbaminsäureester, -thiol-carbaminsäureester, -semicarbazide oder -semicarbazone sein. Vorzugsweise wird die Umsetzung von Benzolsulfonylcarbamidsäureestern mit Aminen benutzt.

Die für die Umsetzung der in 4-Stellung substituierten Benzolsulfonamide verwendeten $R^3$-substituierten Carbaminsäurederivate sind z.B. die entsprechenden Isocyanate, Carbaminsäureester, Thiolcarbaminsäureester oder Carbaminsäurehalogenide. Vorzugsweise verwendet man hiervon die Reaktion der Sulfonamide mit Isocyanaten bzw. Carbaminsäurechloriden.

Die erwähnten Benzolsulfonyl-carbaminsäureester bzw. -thiolcarbaminsäureester können in der Alkoholkomponente einen Alkylrest oder einen Arylrest oder auch einen heterocyclischen Rest aufweisen. Da dieser Rest bei der Reaktion abgespalten wird, hat seine chemische Konstitution keinen Einfluß auf den Charakter des Enproduktes und kann deshalb in weiten Grenzen variiert werden. Das gleiche gilt für die N-$R^3$-substituierten Carbaminsäureester bzw. die entsprechenden Thiolcarbaminsäureester.

Die Spaltung der als Ausgangsstoffe in Verfahren b) genannten Benzolsulfonylparabansäuren, -isoharnstoffäther, -isothioharnstoffäther oder -halogenameisensäureamidine erfolgt zweckmäßig durch alkalische Hydrolyse. Isoharnstoffäther können auch in einem sauren Medium mit gutem Erfolg gespalten werden.

Der Ersatz des Schwefelatoms in der Thioharnstoffgruppierung von entsprechend substituierten Benzolsulfonylthioharnstoffen durch ein Sauerstoffatom kann in bekannter Weise zum Beispiel mit Hilfe von Oxyden oder Salzen von Schwermetallen oder auch durch Anwendung von Oxydationsmitteln, wie Wasserstoffperoxid, Natriumperoxid, salpetriger Säure oder Permanganaten ausgeführt werden. Die Thioharnstoffe können auch entschwefelt werden durch Behandlung mit Phosgen oder Phosphorpentachlorid. Als Zwischenstufe erhaltene Chlorameisensäureamidine bzw. Carbodiimide können durch geeignete Maßnahmen wie Verseifen oder Anlagerung von Wasser in die Benzolsulfonylharnstoffe überführt werden.

Die Oxydation von Benzolsulfinyl- bzw. Benzolsulfenylharnstoffen erfolgt nach an sich bekannter Methode, vorzugsweise mit Oxydationsmitteln wie Permanganat oder Wasserstoffperoxid.

Die Herstellung der Sulfonylharnstoffe gemäß Verfahren e) kann mit reaktiven Derivaten der Säure

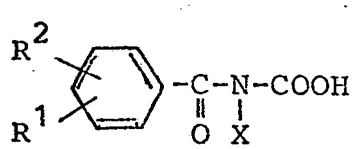

wie beispielsweise Halogeniden, erfolgen. Wenn X Wasserstoff bedeutet, eignen sich besonders gut die entsprechenden Benzoylisocyanate.

Als Sulfonyl- bzw. Sulfinylhalogenide gemäß Verfahren f) eignen sich insbesondere die Chloride. Als saures Kondensationsmittel kann man beispielsweise Thionylchlorid oder Polyphosphonsäure einsetzen.

Die Herstellung der physiologisch verträglichen Salze erfolgt nach an sich bekannten Methoden. Zur Salzbildung sind insbesondere geeignet Alkali- und Erdalkalihydroxyde, -carbonate oder -bicarbonate sowie physiologisch verträgliche organische Basen.

Die Ausführungsformen des Verfahrens gemäß der Erfindung können im allgemeinen hinsichtlich der Reaktionsbedingungen weitgehend variiert und den jeweiligen Verhältnissen angepaßt werden. Beispielsweise können die Umsetzungen in Abwesenheit oder Anwesenheit von Lösungsmitteln, bei Raumtemperatur oder bei erhöhter Temperatur durchgeführt werden.

Je nach dem Charakter der Ausgangsstoffe kann das eine oder andere der beschriebenen Verfahren in einzelnen Fällen einen gewünschten individuellen Benzolsulfonylharnstoff nur in geringen Ausbeuten liefern oder zu dessen Synthese nicht geeignet sein. In solchen verhältnismäßig selten auftretenden Fällen macht es dem Fachmann keine Schwierigkeiten, das gewünschte Produkt auf einem anderen der beschriebenen Verfahrenswege zu synthetisieren.

Die erhaltenen Verbindungen können durch Umfällen und/oder Umkristallisieren gereinigt werden. Die Reinigung kann auch erfolgen, indem man die Substanz aus einem kristallinen (Alkali)-Salz in einem geeigneten Lösungsmittel in Freiheit setzt.

Die erfindungsgemäßen Verbindungen zeichnen sich durch wertvolle pharmakologische Eigenschaften, insbesondere blutzuckersenkende, aus. Sie eignen sich daher als Arzneimittel, insbesondere als Antidiabetika. .

Die blutzuckersenkende Wirkung der erfindungsgemäßen Benzolsulfonylharnstoffe kann dadurch festgestellt werden, daß man sie als freie Verbindungen oder in Form der Natriumsalze an normal ernährte Kaninchen verfüttert und den Blutzuckerwert nach der bekannten Methode von Hagedorn-Jensen oder mit einem Autoanalyzer über eine längere Zeitdauer ermittelt.

Die routinemäßige Bestimmung der blutzuckersenkenden Wirkung kann mit Dosierungen von z.B. 10,2 oder 0,4 mg oder mit noch geringeren Mengen Wirksubstanz pro kg Versuchstier nach bekannten Methoden erfolgen.

Die folgenden Verbindungen I bis V wurden in Dosierungen von 2 mg/kg Kaninchen oral verabreicht und die Blutzuckerwerte wurden mit einem Autoanalyzer über eine Zeitdauer von 24 Stunden ermittelt. Die hierbei gemessene Blutzuckersenkung ist in der nachfolgenden Tabelle in % nach ... Stunden angegeben.

I N-(4-<2-(N'-<5-Chlor-2-methoxy-benzoyl>-ureido)-äthyl>-benzolsulfonyl)-N'-cyclohexyl-harnstoff

II N-(4-<2-(N'-<5-Chlor-2-methoxy-benzoyl>-ureido)-äthyl>-benzolsulfonyl)-N'-butyl-harnsotff

III N-(4-<2-(N'-<5-Chlor-2-methoxy-benzoyl>-ureido)-äthyl>-benzolsulfonyl)-N'-cyclopentyl-harnstoff

IV N-(4-<2-(N'-<2-Methoxy-5-methyl-benzoyl>-ureido)-äthyl>-benzolsulfonyl)-N'-cyclohexyl-harnstoff

V N-(4-<2-(N'-<2-Methoxy-5-methyl-benzoyl>-ureido)-äthyl>-benzolsulfonyl)-N'-(4-methyl-cyclohexyl)-harnstoff

Tabelle

| Verbindung | Blutzuckersenkung am Kaninchen nach Verabreichung von 2 mg/kg p.o. in % nach ... Stunden | | | |
|---|---|---|---|---|
| | 1 | 3 | 6 | 24 |
| I | 24 | 33 | 37 | 23 |
| II | 20 | 33 | 32 | 16 |
| III | 23 | 30 | 31 | 27 |
| IV | 36 | 34 | 43 | 12 |
| V | 38 | 31 | 33 | 11 |

Die Verbindungen I, IV und V wurden in Dosierungen von 0,4 mg/kg oral an Kaninchen verabreicht und die Blutzuckersenkung gemessen. Sie betrug nach 6 Stunden 40 % (Verbindung I), 41 % (Verbindung IV) und 33 % (Verbindung V).

Die erfindungsgemäßen Acylureidoalkylbenzolsulfonylharnstoffe zeichnen sich durch eine starke blutzuckersenkende Wirkung aus. Außerdem sind die Verbindungen gut verträglich.

Die Eigenschaften der Verbindungen erlauben es, in der Therapie des Diabetes mellitus mit so geringen Dosen auszukommen, daß das Präparat nur die verminderte Ansprechbarkeit des Pankreas auf einen erhöhten Blutzuckerspiegel wieder normalisiert.

Benzolsulfonylharnstoffe mit Ureidoalkylrest sind schon mehrfach beschrieben worden (DE-PS 14 43 911, DE-AS 16 70 700, DE-PS 16 18 389, DE-PS 22 38 870, DE-OS 28 28 079). Es war nicht zu erwarten, daß die erfindungsgemäßen Verbindungen sich durch die oben erwähnten günstigen Eigenschaften auszeichnen.

Die beschriebenen Sulfonylharnstoffe sollen vorzugsweise zur Herstellung von oral verabreichbaren Präparaten zur Behandlung des Diabetes mellitus dienen. Sie können als solche oder in Form ihrer Salze bzw. in Gegenwart von Stoffen, die zu einer Salzbildung führen, appliziert werden. Zur Salzbildung können beispielsweise alkalische Mittel wie Alkali-

oder Erdalkalihydroxyde, -carbonate oder -bicarbonate herangezogen werden. Die Präparate können neben dem Sulfonylharnstoff bzw. dessen Salz auch noch andere Wirkstoffe enthalten.

Als medizinische Präparate kommen vorzugsweise Tabletten in Betracht, die neben den Sulfonylharnstoffen oder deren Salzen die üblichen Träger- und Hilfsstoffe wie Talkum, Stärke, Milchzucker oder Magnesiumstearat enthalten. Dabei kann es zweckmäßig sein, den oder die Wirkstoffe in gemahlener oder fein gefällter Form oder als Gemisch dieser Formen einzusetzen. Ein Präparat, das die erfindungsgemäßen Benzolsulfonylharnstoffe als Wirkstoffe enthält, z.B. eine Tablette oder ein Pulver mit oder ohne Zusätze, ist zweckmäßig in eine geeignet dosierte Form gebracht. Als Dosis ist dabei eine solche zu wählen, die der Wirksamkeit des verwendeten Benzolsulfonylharnstoffs und dem gewünschten Effekt angepaßt ist. Zweckmäßig beträgt die Dosierung je Einheit etwa 0,5 bis 50 mg, vorzugsweise 1 bis 20 mg, jedoch können auch darüber oder darunter liegende Dosierungseinheiten verwendet werden, die gegebenenfalls vor Applikation zu teilen bzw. zu vervielfachen sind.

Die nachfolgenden Beispiele zeigen einige der zahlreichen Verfahrensvarianten, die zur Synthese der erfindungsgemäßen Sulfonylharnstoffe verwendet werden können. Sie sollen jedoch nicht eine Einschränkung des Erfindungsgegenstandes darstellen.

Beispiel 1:

N-(4-⟨2-(N'-Benzoylureido)-äthyl⟩-benzolsulfonyl)-N'-cyclohexyl-harnstoff

a. 4-⟨2-(N'-Benzoylureido)-äthyl⟩-benzolsulfonamid
13,4 g N-Benzoyl-N'-(2-phenyläthyl)-harnstoff (Schmp. 137 - 139°C, hergestellt durch Umsetzung von (2-Phenyl-äthyl)-harnstoff mit Benzoylchlorid in Gegenwart von etwas Schwefelsäure) werden in 70 g Chlorsulfonsäure unter Rühren und Eiskühlung eingetragen. Man rührt kurze Zeit nach, erhitzt 1 Stunde auf 50°C und gießt nach Abkühlen auf Eis. Das abgeschiedene Sulfochlorid wird abgesaugt, als Rohprodukt portionsweise in 250 ml konz. Ammoniak eingetragen, das Gemisch etwas erwärmt, gekühlt, das Sulfonamid abgesaugt und aus Methanol-DMF umkristallisiert. Schmp. 247 - 249°C.

b. N-(4-⟨2-(N'-Benzoylureido)-äthyl⟩-benzolsulfonyl)-N'-cyclohexyl-harnstoff

3,5 g Sulfonamid werden in 150 ml Aceton mit 6 g gemahlenem Kaliumcarbonat mehrere Stunden unter Rühren am Rückflußkühler gekocht. Anschließend gibt man 1,3 g Cyclohexylisocyanat zu, erhitzt weitere 6 Stunden unter Rühren zum Sieden, dampft das Aceton unter vermindertem Druck ab, versetzt den Rückstand mit Wasser, filtriert und säuert an. Das abgeschiedene Produkt wird aus verdünntem Ammoniak umgefällt, aus Methanol-Dimethylformamid umkristallisiert und schmilzt bei 200 - 202°C.

Beispiel 2:

N-(4-⟨2-(N'-Benzoyl-N'-methylureido)-äthyl⟩-benzolsulfonyl)-N'-cyclohexyl-harnstoff

13,5 g Benzoesäuremethylamid werden in 250 ml Tetrahydrofuran unter Rühren mit
3,5 g 80proz. Natriumhydrid versetzt und
unter Rühren mehrere Stunden zum Sieden erhitzt. Anschließend wird gekühlt und das Reaktionsgemisch unter Rühren zu einer Lösung von 25 g Phosgen in 125 ml Toluol gegeben. Dabei wird die Temperatur auf etwa 35°C gehalten. Man rührt noch etwas nach, kühlt, saugt ab und engt das Filtrat unter vermindertem Druck ein.
6,8 g des rohen Carbamidsäurechlorid werden in 20 ml Aceton unter Rühren und Kühlung zu einem Gemisch von
5,4 g N-(4-⟨2-Aminoäthyl⟩-benzolsulfonyl-N'-cyclohexylharnstoff in 170 ml Aceton, 170 ml Wasser, 4,4 g Natriumbicarbonat getropft. Man rührt 1 Stunde nach, erhitzt 1 Stunde auf 50°C, engt anschließend unter vermindertem Druck ein, nimmt den Rückstand in Wasser auf, säuert mit verdünnter Essigsäure an, behandelt die Schmiere mit Aceton, filtriert und versetzt das Filtrat mit Wasser. Der ausgefällte N-(4-⟨2-(N'-Benzoyl-N'-methylureido)-äthyl⟩-benzolsulfonyl)-N'-Cyclohexyl -harnstoff wird aus verd. Aceton umkristallisiert und schmilzt bei 177 - 178°C.
In analoger Weise erhält man den
N-(4-⟨2-(N'-Benzoyl-N'-methyl-ureido)-äthyl⟩-benzolsulfonyl)-N'-butyl-harnstoff vom Schmp. 147 - 149°C (aus verd. Aceton),

N-(4-⟨2-(N'-4-Methyl-benzoyl-N'-methyl-ureido)-äthyl⟩-benzolsulfonyl)-N'-cyclohexyl-harnstoff vom Schmp. 200 - 202° (aus verd. Aceton),

N-(-⟨2-(N'-4-Methyl-benzoyl-N'-methyl-ureido)-äthyl⟩-benzolsulfony)-N'-butyl-harnstoff vom Schmp. 167 - 169° (aus verd. Aceton),

N-(4-⟨2-(N'-3-Methyl-benzoyl-N'-methyl-ureido)-äthyl⟩-benzolsulfonyl)-N'-cyclohexyl-harnstoff vom Schmp. 196 - 198° (aus Methanol-Dioxan),

N-(4-⟨2-(N'-3-Methyl-benzoyl-N'-methyl-ureido)-äthyl⟩-benzolsulfonyl)-N'-butyl-harnstoff vom Schmp. 128 - 131° (aus Methanol-Dioxan),

N-N-(4-⟨2-(N'-4-Chlor-benzoyl-N'-methyl-ureido)-äthyl⟩-benzolsulfonyl)-N'-cyclohexyl-harnstoff vom Schmp. 163 - 166° (aus verdünntem Methanol),

N-(4-⟨2-(N'-4-Chlor-benzoyl-N'-methyl-ureido)-äthyl⟩-benzolsulfonyl -N'-butyl-harnstoff vom Schmp. 118 - 122° (aus verd. Methanol),

N-(4-⟨2-(N'-3-Chlor-benzoyl-N'-methyl-ureido)-äthyl⟩-benzolsulfonyl)-N'-cyclohexyl-harnstoff vom Schmp. 168 - 171° (aus verd. Methanol),

N-(4-⟨2-(N'-3-Chlor-benzoyl-N'-methyl-ureido)-äthyl⟩-benzolsulfonyl)-N'-butyl-harnstoff vom Schmp. 140 - 142° (aus verd. Methanol).

BAD ORIGINAL

Beispiel 3:

N-(4-⟨2-(N'-⟨5-Chlor-2-methoxy-benzoyl⟩-ureido)-äthyl⟩-benzolsulfonyl)-N'-cyclohexyl-harnstoff

27 g N-2-Phenäthyl-N'-(5-chlor-2-methoxy-benzoyl)-harnstoff (Schmp. 155 - 157°C, hergestellt aus 5-Chlor-2-methoxy-benzoyl-isocyanat und 2-Phenäthylamin) werden bei 20°C portionsweise in 50 ml Chlorsulfonsäure eingetragen. Anschließend rührt man 3 Stunden bei Raumtemperatur nach, gießt vorsichtig auf Eis, trennt das Sulfochlorid ab und behandelt es mit concentrierter Ammoniak-Lösung. Das Sulfonamid wird abgesaugt und aus Äthanol-Dimethylformamid umkristallisiert. Schmp. 206 - 208°C.

3,1 g 4-(2-⟨N'-(5-Chlor-2-methoxy-benzoyl)-ureido⟩-äthyl)-benzolsulfonamid werden in 50 ml Aceton mit 3,75 ml 2 N-Natronlauge in Lösung gebracht und auf 0 - 5°C abgekühlt. Unter Rühren und Eiskühlung tropft man 1,04 g Cyclohexylisocyanat, gelöst in wenig Aceton, zu und rührt dann 1 Stunde unter Eiskühlung und 2 Stunden bei Raumtemperatur nach. Anschließend setzt man so viel Wasser zu, daß eine Lösung entsteht, säuert mit verdünnter Salzsäure an und saugt ab. Nach dem Umfällen aus verdünnter Ammoniak-Lösung mit verdünnter Salzsäure wird aus Äthanol umkristallisiert. Der so hergestellte N-(4-⟨2-(N'-⟨5-Chlor-2-methoxy-benzoyl⟩-ureido)-äthyl⟩-benzolsulfonyl)-N'-cyclohexyl-harnstoff schmilzt bei 198 - 200°C.

BAD ORIGINAL

In analoger Weise erhält man den

N-(4-⟨2-(N'-⟨5-Chlor-2-methoxy-benzoyl⟩-ureido)-äthyl⟩-benzolsulfonyl)-N'-butyl-harnstoff vom Schmp. 174 - 176°C (aus Äthanol)

In analoger Weise erhält man aus dem 4-Chlor-benzoyl-isocyanat über den

N-2-Phenäthyl-N'-(4-chlor-benzoyl)-harnstoff (Schmp. 178 - 180°C) und das 4-(2-⟨N'-(4-Chlor-benzoyl)-ureido⟩-äthyl)-benzolsulfonamid (Schmp. 226 - 228°C) den

N-(4-⟨2-(N'-⟨4-Chlor-benzoyl⟩-ureido)-äthyl⟩-benzolsulfonyl)-N'-cyclohexyl-harnstoff vom Schmp. 218 - 220°C (aus Äthanol-Dimethylformamid)

N-(4-⟨2-(N'-⟨4-Chlor-benzoyl⟩-ureido)-äthyl⟩-benzolsulfonyl)-N'-(4-methyl-cyclohexyl)-harnstoff vom Schmp. 242 - 244°C (aus Äthanol-Dimethylformamid)

N-(4-⟨2-(N'-⟨4-Chlor-benzoyl⟩-ureido)-äthyl⟩-benzolsulfonyl)-N'-butyl-harnstoff vom Schmp. 188 - 190°C (aus Äthanol-Dimethylformamid)

BAD ORIGINAL

In analoger Weise erhält man aus dem 4-Methyl-benzoyl-isocyanat über den

N-2-Phenäthyl-N´-(4-methyl-benzoyl)-harnstoff (Schmp. 156 - 158 °C) und das 4-(2-⟨N´-(4-Methyl-benzoyl)-ureido⟩-äthyl)-benzolsulfonamid (Schmp. 239 - 241 °C) den

N-(4-⟨2-(N´-⟨4-Methyl-benzoyl⟩-ureido)-äthyl⟩-benzolsulfonyl)-N´-cyclohexyl-harnstoff vom Schmp. 219 - 221 °C (aus Äthanol-Dimethylformamid)

N-(4-⟨2-(N´-⟨4-Methyl-benzoyl⟩-ureido)-äthyl⟩-benzolsulfonyl)-N´-butyl-harnstoff vom Schmp. 208 - 210 °C (aus Äthanol-Dimethylformamid)

BAD ORIGINAL

Beispiel 4:

N-(4-⟨2-(N'-⟨2-Methoxy-5-methyl-benzoyl⟩-ureido)-äthyl⟩-benzolsulfonyl)-N'-cyclohexyl-harnstoff

31,2 g N-2-Phenäthyl-N'-(2-methoxy-5-methyl-benzoyl)-harnstoff (Schmp. 123 - 125°C, hergestellt aus 2-Methoxy-5-methyl-benzoyl-isocyanat und 2-Phenäthylamin) werden in 400 ml Chloroform gelöst und auf 0 - 5°C abgekühlt. Unter Rühren tropft man bei Raumtemperatur 50 ml Chlorsulfonsäure zu und rührt dann 3 Stunden bei Raumtemperatur nach. Man gießt vorsichtig auf Eiswasser, trennt die organische Phase ab, engt ein und behandelt den Rückstand mit concentrierter Ammoniak-Lösung. Das Sulfonamid wird abgesaugt und aus Äthanol-Dimethylformamid umkristallisiert.
Schmp. 204 - 206°C.

2,93 g 4-(2-⟨N'-(2-Methoxy-5-methyl-benzoyl)-ureido⟩-äthyl)-benzolsulfonamid werden in 150 ml 2-Butanon zusammen mit 2,07 g gemahlener Pottasche und 1,04 g Cyclohexylisocyanat vier Stunden unter Rückfluß gerührt. Nach dem Erkalten wird filtriert, das Salzgemisch in Wasser gelöst und mit 2 N-Salzsäure angesäuert. Der Niederschlag wird abgesaugt, aus verdünnter Ammoniak-Lösung mit verdünnter Salzsäure umgefällt und aus Nitromethan umkristallisiert. Der Schmelzpunkt des N-(4-⟨2-(N'-⟨2-Methoxy-5-methyl-benzoyl⟩-ureido)-äthyl⟩-benzolsulfonyl)-N'-cyclohexyl-harnstoff liegt bei 138 - 140°C.

BAD ORIGINAL

In analoger Weise erhält man den

N-(4-⟨2-(N′-⟨2-Methoxy-5-methyl-benzoyl⟩-ureido)-äthyl⟩-benzolsulfonyl)-N′-(4-methyl-cyclohexyl)-harnstoff
vom Schmp. 188 - 190°C (aus Äthanol)

N-(4-⟨2-(N′-⟨2-Methoxy-5-methyl-benzoyl⟩-ureido)-äthyl⟩-benzolsulfonyl)-N′-butyl-harnstoff
vom Schmp. 170 - 172°C (aus Äthanol)

In analoger Weise erhält man aus dem 3,4-Dichlor-benzoylisocyanat über den

N-2-Phenäthyl-N′-(3,4-dichlor-benzoyl)-harnstoff (Schmp. 160 - 162°C) und das 4-(2-⟨N′-(3,4-Dichlor-benzoyl)-ureido⟩-äthyl)-benzolsulfonamid den

N-(4-⟨2-(N′-⟨3,4-Dichlor-benzoyl⟩-ureido)-äthyl⟩-benzolsulfonyl)-N′-cyclohexyl-harnstoff
vom Schmp. 215 - 217°C (aus Äthanol-Dimethylformamid)

N-(4-⟨2-(N′-⟨3,4-Dichlor-benzoyl⟩-ureido)-äthyl⟩-benzolsulfonyl)-N′-(4-methyl-cyclohexyl)-harnstoff
vom Schmp. 225 - 227°C (aus Dimethylformamid-Wasser)

BAD ORIGINAL

N-(4-⟨2-(N´-⟨3,4-Dichlor-benzoyl⟩-ureido)-äthyl⟩-benzolsulfonyl)-N´-butyl-harnstoff vom Schmp. 180 - 182°C (aus Dimethylformamid-Wasser)

In analoger Weise erhält man aus dem 2-Methyl-benzoyl-isocyanat über den

N-2-Phenäthyl-N´-(2-methyl-benzoyl)-harnstoff (Schmp. 132 - 133°C) und das 4-(2-⟨N´-(2-Methyl-benzoyl)-ureido⟩-äthyl)-benzolsulfonamid (Schmp. 236 - 238°C) den

N-(4-⟨2-(N´-⟨2-Methyl-benzoyl⟩-ureido)-äthyl⟩-benzolsulfonyl)-N´-cyclohexyl-harnstoff vom Schmp. 182 - 184°C (aus Äthanol-Dimethylformamid)

N-(4-⟨2-(N´-⟨2-Methyl-benzoyl⟩-ureido)-äthyl⟩-benzolsulfonyl)-N´-(4-methyl-cyclohexyl)-harnstoff vom Schmp. 208 - 210°C (aus Äthanol)

In analoger Weise erhält man aus dem 3-Methyl-benzoyl-isocyanat über den

N-2-Phenäthyl-N´-(3-methyl-benzoyl)-harnstoff (Schmp. 90 - 92°C) und das 4-(2-⟨N´-(3-Methyl-benzoyl)-ureido⟩-äthyl)-benzolsulfonamid (Schmp. 231 - 233°C) den

BAD ORIGINAL

N-(4-<2-(N'-<3-Methyl-benzoyl>-ureido)-äthyl>-benzolsulfonyl)-N'-cyclohexyl-harnstoff
vom Schmp. 212 - 213°C (aus Äthanol-Dimethylformamid)

N-(4-<2-(N'-<3-Methyl-benzoyl>-ureido)-äthyl>-benzolsulfonyl)-N'-(4-methyl-cyclohexyl)-harnstoff
vom Schmp. 214 - 215°C (aus Äthanol-Dimethylformamid)

In analoger Weise erhält man aus dem 4-(2-<N'-(5-Chlor-2-methoxybenzoyl)-ureido>-äthyl)-benzolsulfonamid (Herstellung siehe Beispiel 3) den
N-(4-<2-(N'-<5-Chlor-2-methoxy-benzoyl>-ureido)-äthyl>-benzol-sulfonyl)-N'-cyclopentylmethylharnstoff
vom Schmp. 197 - 199°C (aus Äthanol/DMF)

N-(4-<2-(N'-<5-Chlor-2-methoxy-benzoyl>-ureido)-äthyl>-benzolsulfonyl)-N'-3-methyl-cyclopentylmethylharnstoff
vom Schmp. 184 - 186°C (aus Nitromethan)

N-(4-<2-(N'-<5-Chlor-2-methoxy-benzoyl>-ureido)-äthyl>-benzolsulfonyl)-N'-2,5-endomethylen-cyclohexylmethyl-harnstoff
vom Schmp. 170 - 172°C (aus Äthanol)

N-(4-<2-(N'-<5-Chlor-2-methoxy-benzoyl>-ureido)-äthyl>-benzol-sulfonyl)-N'-4-äthylcyclohexyl-harnstoff
vom Schmp. 185°C (aus Nitromethan)

N-(4-<2-(N'-<5-Chlor-2-methoxy-benzoyl>-ureido)-äthyl>-benzol-sulfonyl)-N'-$\Delta^3$-cyclohexenyl-harnstoff
vom Schmp. 105°C (aus Chloroform)

N-(4-<2-(N'-<5-Chlor-2-methoxy-benzoyl>-ureido)-äthyl>-benzol-sulfonyl)-N'-propyl-harnstoff
vom Schmp. 176 - 177°C (aus Äthanol)

BAD ORIGINAL

N-(4-(2-(N'-(5-Chlor-2-methoxy-benzoyl)-ureido)-äthyl)-benzolsulfonyl)-N'-benzyl-harnstoff
vom Schmp. 193 - 195°C (aus Äthanol/DMF)

N-(4-(2-(N'-(5-Chlor-2-methoxy-benzoyl)-ureido)-äthyl)-benzolsulfonyl)-N'-cyclopentyl-harnstoff
vom Schmp. 185 - 187°C (aus Chloroform)

N-(4-(2-(N'-(5-Chlor-2-methoxy-benzoyl)-ureido)-äthyl)-benzolsulfonyl)-N'-cycloheptyl-harnstoff
vom Schmp. 187 - 188°C (aus Nitromethan)

N-(4-(2-(N'-(5-Chlor-2-methoxy-benzoyl)-ureido)-äthyl)-benzolsulfonyl)-N'-4-methylcyclohexyl-harnstoff
vom Schmp. 165 - 167°C (Rohprodukt)

In analoger Weise erhält man aus dem 2,5-Dimethyl-benzoyl-isocyanat über den
N-2-Phenäthyl-N'-(2,5-dimethyl-benzoyl)-harnstoff
(Schmp. 234 - 236°C) den

N-(4-(2-(N'-(2,5-Dimethyl-benzoyl)-ureido)-äthyl)-benzolsulfonyl)-N'-cyclohexyl-harnstoff
vom Schmp. 211 - 213°C (aus Äthanol/DMF)

N-(4-(2-(N'-(2,5-Dimethyl-benzoyl)-ureido)-äthyl)-benzolsulfonyl)-N'-butyl-harnstoff
vom Schmp. 197- 199°C (aus Äthanol/DMF)

In analoger Weise erhält man aus dem 2-Propyloxy-benzoyl-isocyanat über den
N-2-Phenäthyl-N'-(2-propyloxy-benzoyl)-harnstoff
vom Schmp. 168 - 170°C den

N-(4-(2-(N'-(2-Propyloxy-benzoyl)-ureido)-äthyl)-benzolsulfonyl)-N'-cyclohexyl-harnstoff
vom Schmp. 174 - 176°C (aus Äthanol)

BAD ORIGINAL

N-(4-<2-(N'-<2-Propyloxy-benzoyl>-ureido)-äthyl>-benzolsulfonyl)-N'-4-methylcyclohexyl-harnstoff
vom Schmp. 184 - 186°C (aus Äthanol)

N-(4-<2-(N'-<2-Propyloxy-benzoyl>-ureido)-äthyl>-benzolsulfonyl)-N'-butyl-harnstoff
vom Schmp. 152 - 154°C (aus Äthanol)

Beispiel 5:

N-(4-<2-(N'-(5-Chlor-2-methoxy-benzoyl>-ureido)-äthyl>-benzolsulfonyl)-N'-hexyl-harnstoff

1. 4-(2-<N'-(5-Chlor-2-methoxy-benzoyl)-ureido>-äthyl)-benzolsulfonylcarbaminsäuremethylester
   4,11 g 4-(2-<N'-(5-Chlor-2-methoxy-benzoyl)-ureido>-äthyl)-benzolsulfonamid und 2,76 g $K_2CO_3$ (gemahlen) werden in 150 ml Butanon-(2) 4 h unter Rückfluß gerührt. Man setzt dann 1,41 g Chlorameisensäuremethylester zu und rührt weitere 4 h unter Rückfluß nach. Die Suspension wird anschließend i.V. eingeengt und der Rückstand in verd. Ammoniak gelöst. Man saugt durch eine Seitz-Kohle-Schicht und säuert mit 2n HCl an. Das Urethan wird abgesaugt und aus $CH_3OH/H_2O$ umkristallisiert. Fp: 178 - 180.

2. N-(4-<2-(N'-(5-Chlor-2-methoxy-benzoyl>-ureido)-äthyl>-benzolsulfonyl)-N'-hexyl-harnstoff

   2,34 g Urethan werden mit 1,03 g Hexylamin in 60 ml Dioxan 5 h unter Rückfluß gerührt. Die Lösung wird i.V. eingeengt und der Rückstand in Wasser gelöst. Man säuert mit 2n HCl an

BAD ORIGINAL

0047496

und extrahiert dann den öligen Niederschlag mit $CHCl_3$. Die org. Phase wird über $Na_2SO_4$ getrocknet und wieder eingeengt. Der Harnstoff wird aus Essigester/EtOH umkristallisiert.
Fp. 173 - 175°.

In analoger Weise erhält man den N-(4-<2-(N'-<5-Chlor-2-methoxy-benzoyl>-ureido)-äthyl>-benzolsulfonyl)-N'-4-methoxycyclohexyl-harnstoff vom Schmp. 115 - 117°C (mit Äther verrieben)

N-(4-<2-(N'-<5-Chlor-2-methoxy-benzoyl>-ureido)-äthyl>-benzolsulfonyl)-N'-(2,5-endomethylen-cyclohexyl)-harnstoff vom Schmp. 183 - 185°C (aus Nitromethan)

BAD ORIGINAL

## Beispiel 6:

**N-(4-(2-(N'-(2-Propyloxy-benzoyl)-ureido)-äthyl)benzolsulfonyl)-N'-cyclohexylharnstoff**

1,64 g N-(4-(2-(N'-(2-Propyloxy-benzoyl)-ureido)-äthyl)-benzolsulfonyl)-N'-cyclohexyl-thioharnstoff (Schmp. 146 - 148°C, hergestellt aus 4-(2-(N'-(2-Propyloxy-benzoyl)-ureido)-äthyl)-benzolsulfonamid und Cyclohexylsenföl) werden in 60 ml Methanol nach Zusatz von 0,63 g gelbem Quecksilberoxid 6 Stunden bei 50 - 55°C gerührt. Nach dem Abkühlen saugt man das gebildete Quecksilbersulfid ab und engt im Vakuum ein. Der Rückstand wird nach Zusatz von Äther fest und schmilzt bei 118 - 120°C.

0,81 g des so erhaltenen N-(4-(2-(N'-(2-Propyloxybenzoyl)-ureido)-äthyl)-benzolsulfonyl)-N'-cyclohexyl-isoharnstoff-methyläthers werden in 25 ml conc. Salzsäure 4 Stunden bei 40 - 50°C gerührt. Nach dem Verdünnen mit 50 ml Wasser saugt man ab und kristallisiert aus Äthanol um.
Der so erhaltene N-(4-(2-(N'-(2-Propyloxy-benzoyl)-ureido)-äthyl)-benzolsulfonyl)-N'-cyclohexylharnstoff schmilzt bei 174 - 176°C.

BAD ORIGINAL

Beispiel 7:

N-(4-(2-(N'-(2-Propyloxy-benzoyl)-ureido)-äthyl)-benzolsulfonyl)-N'-cyclohexyl-harnstoff

1,2 g N-(4-(2-(N'-(2-Propyloxy-benzoyl)-ureido)-äthyl)-benzolsulfonyl)-N'-cyclohexylthioharnstoff (Schmp. 146 - 148°C, hergestellt aus 4-(2-(N'-(2-Propyloxy-benzoyl)ureido)-äthyl)-benzolsulfonamid und Cyclohexylsenföl) werden in 10 ml Methanol und 30 ml Wasser nach Zugabe von 0,5 g gelbem Quecksilberoxid 8 Stunden bei 60°C gerührt. Nach dem Abkühlen filtriert man das Quecksilbersulfid ab, dampft im Vakuum ein, fällt den Rückstand aus verdünnter Ammoniak-Lösung mit verdünnter Salzsäure um und kristallisiert schließlich den N-(4-(2-(N'-(2-Propyloxy-benzoyl)-ureido)-äthyl)-benzolsulfonyl)-N'-cyclohexylharnstoff aus Äthanol um. Er schmilzt bei 174 - 176°C.

Patentansprüche:

1. Sulfonylharnstoffe der Formel

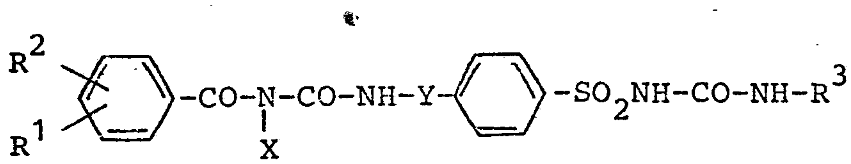

in welcher bedeuten:

$R^1$, $R^2$ Wasserstoff, Alkyl, Alkoxy mit jeweils 1 - 3 C-Atomen im Alkylteil, oder Halogen, wobei $R^1$ und $R^2$ gleich oder verschieden sein können,

X Wasserstoff, Alkyl mit 1 - 3 C-Atomen,

Y Alkylen mit 2 - 3 C-Atomen

$R^3$ Alkyl von 3 - 8 C-Atomen, Cycloalkyl, Alkylcycloalkyl, Dialkylcycloalkyl, Cycloalkylalkyl, Cycloalkenyl, Alkylcycloalkenyl mit jeweils 4 - 9 C-Atomen, Methylcyclopentylmethyl, Cyclohexenylmethyl, Chlorcyclohexyl, Methoxycyclohexyl, Bicycloheptyl, Bicycloheptenyl, Bicycloheptylmethyl, Bicycloheptenylmethyl, Bicyclooctyl, Nortricyclyl, Adamantyl oder Benzyl, und deren physiologisch verträgliche Salze.

2. Sulfonylharnstoff gemäß Anspruch 1, dadurch gekennzeichnet, daß $R^1$ Methoxy in 2-Stellung und $R^2$ Chlor oder Methyl in 5-Stellung zur CO-Gruppe, X Wasserstoff, Y die $-CH_2-CH_2-$Gruppe und $R^3$ Butyl, Cyclopentyl, Cyclohexyl oder Methylcyclohexyl bedeutet.

3. Verfahren zur Herstellung von Sulfonylharnstoffen gemäß Anspruch 1, dadurch gekennzeichnet, daß man

a) mit der Gruppe

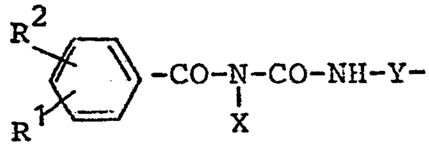

in 4-Stellung substituierte Benzolsulfonyl-carbaminsäurederivate mit einem Amin $R^3-NH_2$ oder dessen Salzen umsetzt oder Sulfonamide der Formel

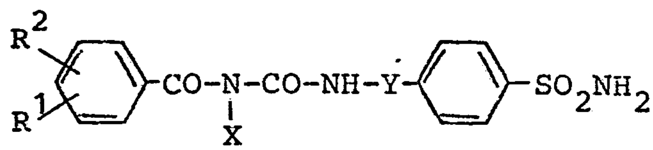

oder deren Salze mit einem $R^3$-substituierten Carbaminsäurederivat umsetzt,

b) mit der Gruppe

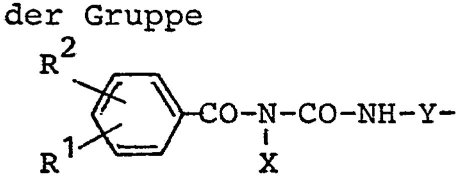

substituierte Benzolsulfonyl-isoharnstoffäther, -isothioharnstoffäther, -parabansäuren oder -halogenameisensäureamidine spaltet,

c) in

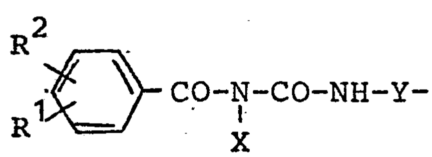

substituierten Benzolsulfonylthioharnstoffen das Schwefelatom durch Sauerstoff ersetzt,

d) entsprechende Benzolsulfinyl- oder sulfenyl-harnstoffe oxydiert,

e) in Benzolsulfonylharnstoffe der Formel

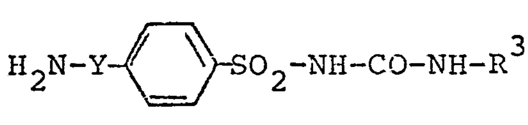

gegebenenfalls stufenweise den Rest

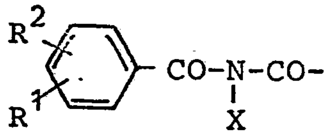

einführt, oder

f) entsprechend substituierte Benzolsulfonylhalogenide mit $R^3$-substituierten Harnstoffen oder deren Alkalisalzen umsetzt oder entsprechend substituierte Benzolsulfinsäure-halogenide oder, in Gegenwart von sauren Kondensationsmitteln, auch entsprechend substituierte Sulfinsäuren oder deren Alkalisalze, mit N-$R^3$-N'-hydroxy-harnstoff umsetzt und die Reaktionsprodukte gegebenenfalls zur Salzbildung mit alkalischen Mitteln behandelt.

4. Arzneimittel auf Basis eines Sulfonylharnstoffs gemäß Anspruch 1 oder eines seiner Salze.

5. Verwendung eines Sulfonylharnstoffs gemäß Anspruch 1 oder eines seiner Salze bei der Bekämpfung von Diabetes.

6. Verfahren zur Herstellung eines Arzneimittels gemäß Anspruch 3, dadurch gekennzeichnet, daß man einen Sulfonylharnstoff der im Anspruch 1 angegebenen Formel oder eines seiner Salze in eine geeignete Applikationsform bringt.

7. Verfahren zur Behandlung des Diabetes mellitus, dadurch gekennzeichnet, daß man eine wirksame Menge einer Verbindung gemäß Anspruch 1 verabreicht.

0047496

Nummer der Anmeldung

Europäisches Patentamt

## EUROPÄISCHER TEILRECHERCHENBERICHT,

der nach Regel 45 des Europäischen Patentübereinkommens für das weitere Verfahren als europäischer Recherchenbericht gilt

EP 81106883.2

| EINSCHLÄGIGE DOKUMENTE | | | KLASSIFIKATION DER ANMELDUNG (Int. Cl.[3]) |
|---|---|---|---|
| Kategorie | Kennzeichnung des Dokuments mit Angabe, soweit erforderlich, der maßgeblichen Teile | betrifft Anspruch | |
| | DE - A - 2 230 543 (FARBWERKE HOECHST)<br>* Ansprüche; Seiten 2-6 *<br>---- | 1,3,6 | C 07 C 143/833<br>C 07 C 139/00<br>A 61 K 31/64 |
| | | | RECHERCHIERTE SACHGEBIETE (Int. Cl.[3]) |
| | | | C 07 C 143/00<br>A 61 K 31/00<br>C 07 C 139/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-4,6

Unvollständig recherchierte Patentansprüche: –

Nicht recherchierte Patentansprüche: 5,7

Grund für die Beschränkung der Recherche:

Verfahren zur therapeutischen Behandlung des menschlichen oder tierischen Körpers Artikel 52(4) EPÜ

KATEGORIE DER GENANNTEN DOKUMENTE

- X: von besonderer Bedeutung
- A: technologischer Hintergrund
- O: nichtschriftliche Offenbarung
- P: Zwischenliteratur
- T: der Erfindung zugrunde liegende Theorien oder Grundsätze
- E: kollidierende Anmeldung
- D: in der Anmeldung angeführtes Dokument
- L: aus andern Gründen angeführtes Dokument
- &: Mitglied der gleichen Patentfamilie, übereinstimmendes Dokument

| Recherchenort | Abschlußdatum der Recherche | Prüfer |
|---|---|---|
| WIEN | 19-11-1981 | TENGLER |